(19) Europäisches Patentamt
European Patent Office
Office européen des brevets

(11) EP 0 666 249 B1

(12) **FASCICULE DE BREVET EUROPEEN**

(45) Date de publication et mention de la délivrance du brevet: **05.03.1997 Bulletin 1997/10**

(51) Int. Cl.[6]: **C07C 5/27**

(21) Numéro de dépôt: **95400195.4**

(22) Date de dépôt: **30.01.1995**

(54) **Procédé d'isomérisation de n-paraffines en isoparaffines**

Verfahren zu Isomerisierung von n-Paraffinen in Isoparaffinen

Process for the isomerisation of n-paraffins to isoparaffins

(84) Etats contractants désignés:
**AT BE CH DE DK ES GB GR IE IT LI LU NL PT SE**

(30) Priorité: **08.02.1994 FR 9401400**

(43) Date de publication de la demande:
**09.08.1995 Bulletin 1995/32**

(73) Titulaire: **TOTAL RAFFINAGE DISTRIBUTION S.A.**
**92800 Puteaux (FR)**

(72) Inventeurs:
- **Mariette, Laurent**
  **F-14800 Deauville (FR)**
- **Fersing, Marc**
  **F-76310 Sainte Adresse (FR)**
- **Laborde, Michel**
  **F-76620 Le Havre (FR)**
- **Couillard, Jaques**
  **F-76620 Le Havre (FR)**

(74) Mandataire: **Jolly, Jean-Pierre et al**
**Cabinet Jolly**
**54, rue de Clichy**
**75009 Paris (FR)**

(56) Documents cités:
**FR-A- 1 546 658**
**US-A- 3 791 960**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Rank Xerox (UK) Business Services
2.13.16/3.4

## Description

La présente invention concerne un procédé d'isomérisation de n-paraffines en isoparaffines.

Pour réaliser de telles réactions d'isomérisation, on peut utiliser, de façon connue, des catalyseurs comprenant un support contenant une zéolite, sur laquelle sont déposés du platine et éventuellement un second métal, choisi, par exemple, dans le groupe constitué par le zirconium, le titane, le molybdène et le tungstène (voir EP-A-0 253 743).

A ces catalyseurs dits "zéolitiques", qui conduisent à des températures de réaction relativement élevées, on préfère souvent des catalyseurs dits "amorphes", qui comprennent un support solide stable, généralement de l'alumine, du platine et, éventuellement, au moins un métal choisi dans le groupe comprenant l'étain, le nickel, le germanium, le rhénium, le plomb ou des métaux des groupes IB, I'B, VB, VIIB, III et IV de la classification périodique des éléments, ces catalyseurs présentant en outre des sites de Lewis du type halogénure métallique et des sites de Brönstedt (voir FR-A- 2 649 989). De tels catalyseurs amorphes permettent, en effet, de conduire les réactions d'isomérisation à des températures nettement plus basses (entre 100 et 200°C) et ils permettent d'obtenir des isomérats proches de l'équilibre thermodynamique.

Ces catalyseurs présentent toutefois des inconvénients majeurs :

- ils sont coûteux et longs à fabriquer ;
- ils sont sensibles à divers composés (oxygène, eau, hydrogène sulfuré, oléfines, hydrocarbures aromatiques etc.), qui les rendent inactifs ; en particulier, les sites de Lewis et de Brönstedt sont détruits par l'eau, en générant de l'acide chlorhydrique, et, dans ce cas, le catalyseur n'est pas régénérable ;
- enfin, leur présence en quantité importante dans un réacteur (généralement de 5 à le plus souvent 50 tonnes) les rend difficiles à surveiller et à contrôler, étant donné leur caractère agressif.

US-A-3 791 960 décrit un procédé d'isomérisation d'hydrocarbures paraffiniques ayant une température d'ébullition dans le domaine des essences, dans lequel on utilise un catalyseur comprenant un métal de la mine de platine, un support d'oxyde réfractaire, et du chlore présent en des sites actifs d'isomérisation. La charge, qui contient une proportion minimum de composés aromatiques, passe d'abord dans une zone d'hydrogénation comprenant un tel catalyseur et opérant à une température de 150 à 350°C, en présence d'un gaz de recycle riche en hydrogène, qui contient du chlorure d'hydrogène, en vue d'hydrogéner les composés aromatiques. L'effluent de cette zone d'hydrogénation est ensuite refroidi et traité dans une zone d'isomérisation à 100-204°C, tandis qu'un composé apte à produire du chlorure d'hydrogène est introduit dans le système entre la zone d'hydrogénation et la zone d'isomérisation. L'effluent de la zone d'isomérisation est traité de manière à séparer un gaz de recycle riche en hydrogène, contenant du chlorure d'hydrogène, qui est recyclé à la zone d'hydrogénation.

Pour mesurer cette conversion en composés à chaîne ramifiée, on définit le taux d'isomérisation, qui peut être mesuré par chromatographie sur les effluents du réacteur, ou par tout autre moyen analytique à la portée de l'homme du métier, à savoir le rayonnement infra-rouge, la spectroscopie de masse ou la résonance magnétique nucléaire.

Pour l'isomérisation des coupes $C_4$, qui comportent moins de composés susceptibles d'empoisonner le catalyseur que les coupes $C_5$-$C_6$, on utilisé généralement un réacteur unique et le taux d'isomérisation est le rapport

$$\frac{\text{i-butane}}{\text{n-butane + i-butane}}.$$

Pour l'isomérisation des coupes $C_5$ et $C_6$ dans le but d'accroître l'indice d'octane de la charge, le taux d'isomérisation est défini par

$$\frac{\text{i pentane}}{\text{pentane}} + \frac{\text{2-2-diméthylbutane}}{\text{hexane}},$$

et il est également possible d'appréhender la conversion en composés à chaîne ramifiée par l'indice d'octane moteur (MON en anglais) mesuré par chromatographie. En munissant les réacteurs d'un catalyseur neuf approprié, il est possible, grâce aux systèmes d'isomérisation, en partant d'une charge paraffinique en $C_5$-$C_6$ d'un MON de l'ordre de 60, d'obtenir un MON de l'ordre de 80 à la sortie du premier réacteur et de l'ordre de 81 à la sortie du second réacteur.

En raffinerie, il est naturellement important de maintenir à un niveau aussi élevé que possible, dans les effluents, le taux d'isomérisation pour les hydrocarbures en $C_4$ et, dans le cas des coupes $C_5$-$C_6$, soit le MON, soit le taux d'isomérisation, car toute chute de ces indices se traduit par une perte économique. Une corrélation peut être faite entre le taux d'isomérisation et le MON, sachant que la variation du taux d'isomérisation est de 0,1 à 30% et la variation du MON est de 0,01 à 3%.

Dans la pratique des réacteurs de raffinerie, on ne peut bien évidemment pas contrôler l'état du catalyseur in situ, étant donné la masse du catalyseur (de l'ordre de 10 à plus de 50 tonnes), le volume du réacteur (plusieurs mètres de diamètre), le caractère corrosif et agressif du milieu et les risques non négligeables de désactivation de la masse catalytique lors de l'ouverture du réacteur. On se contente donc de suivre en continu l'évolution du taux d'isomérisation de la charge traitée (ou d'un paramètre directement lié à ce taux, par exemple l'indice d'octane) dans les effluents. Dès que cet indice a substantiellement diminué, les pertes d'exploitation, liées à la diminution de quantités importantes d'isomérat, rendent évidemment nécessaire le remplacement du catalyseur usé quel qu'en soit l'état par du catalyseur neuf.

Le déchargement du réacteur se fait donc sans précautions particulières, puisque le catalyseur n'est pas réutilisable.

Afin d'optimiser l'utilisation de la masse catalytique non régénérable, et surtout lorsque le besoin en isomérat est particulièrement fort, ce qui est généralement le cas, dans les pays européens, pour les essences à haut indice d'octane, on utilise souvent deux réacteurs d'isomérisation placés en série : la masse catalytique du réacteur placé en aval corrige alors partiellement la baisse d'isomérisation des effluents du réacteur amont.

Néanmoins, même dans cette configuration en série des réacteurs, dès que le réacteur amont a subi une désactivation notable, le raffineur doit choisir entre deux moindres maux :

- ou bien le besoin en isomérat est tel que, dès que la teneur en isomérat des effluents du réacteur aval commence à chuter, il convient de changer préventivement la masse catalytique en amont quelle qu'en soit l'activité résiduelle ;
- ou bien l'on pousse la réaction d'isomérisation du réacteur amont jusqu'à la désactivation complète de la masse catalytique amont, mais on induit alors des pertes d'exploitation par suite de la baisse d'activité du catalyseur dans le deuxième réacteur, qui se concrétise par une baisse du taux d'isomérisation de ce dernier.

L'invention vise donc à remédier aux inconvénients ci-dessus mentionnés pour les unités à un ou deux réacteurs utilisant un catalyseur amorphe présentant des sites de Lewis du type halogénure métallique et/ou des sites de Brönstedt, en proposant un procédé d'isomérisation d'hydrocarbures paraffiniques qui permette de diminuer la consommation en catalyseur du ou des réacteurs, tout en maintenant maximum le taux d'isomérisation des effluents de l'unité.

La Demanderesse a en effet établi que, dans la mesure où toutes les précautions sont prises pour favoriser un écoulement homogène des fluides sur toute la section du ou des réacteurs, contrairement aux sites plus ou moins désactivés par les poisons de la réaction d'isomérisation, les sites détruits par les simples traces d'eau-véhiculées par les fluides réactionnels ne sont pas répartis simultanément en tout point de la masse catalytique, mais au contraire sont placés uniquement dans la partie amont du lit catalytique.

La Demanderesse a pu ainsi démontrer que, de façon surprenante, il existait une corrélation entre le taux d'isomérisation des effluents d'un réacteur et la quantité de sites détruits dans la partie amont dudit réacteur d'isomérisation. Il est donc possible, sans arrêter le réacteur pour effectuer des sondages aussi difficiles que coûteux et sans attendre que la totalité de la masse catalytique ait été complètement épuisée par destruction des sites, de connaître globalement l'état du lit catalytique et d'optimiser l'utilisation du catalyseur, en remplaçant une fraction usée de la partie amont du lit catalytique, lorsque diminue le taux d'isomérisation des effluents du réacteur.

Selon un mode particulièrement avantageux de mise en oeuvre de l'invention et lorsque l'installation le permet, il est également souhaitable d'inverser de temps à autre le sens de circulation des fluides dans le réacteur.

L'invention a par conséquent pour objet un procédé d'isomérisation de n-paraffines en isoparaffines d'une coupe d'hydrocarbures comprenant essentiellement soit des hydrocarbures à quatre atomes de carbone, soit des hydrocarbures à cinq et/ou six atomes de carbone, procédé dans lequel la coupe à traiter passe dans au moins un réacteur contenant un lit fixe de catalyseur que traverse la charge, ce catalyseur comprenant un support en oxyde minéral réfractaire, de 0,1 à 0,25 % en poids d'un métal de la mine du platine et de 2 à 10 % en poids de chlore, la réaction d'isomérisation étant conduite à une température de 100 à 200°C, sous une pression de 7 à 60 bars (7 à $60x10^5$ Pascal), avec une vitesse spatiale horaire (volume de charge par volume de catalyseur et par heure) comprise entre 0,5 et 12, ce procédé étant caractérisé en ce que :

- on mesure, de préférence en continu, le taux d'isomérisation des effluents de ce réacteur ou un paramètre directement lié à ce taux d'isomérisation ;
- lorsque ce taux d'isomérisation a baissé d'une valeur prédéterminée, comprise entre 10 et 30%, on remplace par du catalyseur neuf ou régénéré une fraction disposée dans la partie amont de la masse catalytique du réacteur, représentant entre un tiers et deux tiers de cette masse catalytique ;
- lorsque l'opération de remplacement de la fraction amont du catalyseur du réacteur est terminée, on fait à nouveau traverser ce réacteur à la charge.

Dans la pratique, on mesurera respectivement en continu soit directement le taux d'isomérisation des effluents du

réacteur, par chromatographie, dans le cas d'une charge en $C_4$, soit l'indice d'octane moteur de ces effluents, dans le cas d'une charge en $C_5$-$C_6$.

Selon un mode de mise en oeuvre préféré de l'invention, on inverse périodiquement le sens de circulation après remplacement du catalyseur. Cette inversion du sens de circulation de la charge peut être effectué après chaque remplacement du catalyseur, mais, généralement, on inversera périodiquement le sens de la circulation, mais non après chaque chargement.

L'invention est fondée sur le fait qu'il existe une corrélation assez précise entre la chute du taux d'isomérisation à la sortie du réacteur et la masse catalytique usée à remplacer, disposée en amont de ce réacteur, comme le montre le Tableau suivant :

| Chute du taux d'isomérisation, en % | Fraction amont de la masse catalytique à remplacer en % |
|---|---|
| 10 % | 33 % |
| 15-20 % | 50 % |
| 30 % | 66 à 75 % |

On peut donc dire que, lorsque le taux d'isomérisation des effluents du réacteur a baissé d'une valeur comprise en 10 % et 30 % de sa valeur initiale, et sans nécessairement recourir à des techniques de sondages du lit catalytique telles que décrites dans le brevet FR-A- 2 566 530 déposé par la Demanderesse, il est nécessaire de remplacer une fraction du catalyseur de ce réacteur disposée en amont représentant entre un tiers et deux tiers de la masse catalytique totale de ce réacteur.

Le phénomène de progression d'un front de désactivation des sites actifs du catalyseur est d'autant plus manifeste que le réacteur possède dans la zone d'injection de la charge des moyens de diffusion permettant de répartir les fluides réactionnels de façon aussi homogène que possible sur toute la section réactionnelle, et que le réacteur a été empli de façon homogène en y déversant le catalyseur suivant un flux fin et homogène tombant à la manière d'une pluie suivant toute la section du réacteur (méthode communément appelée méthode a chargement dense), comme décrit par exemple dans FR-A- 2 625 509. Le lit catalytique étant homogène, l'écoulement des fluides se fait alors de façon homogène sur toute la section réactionnelle et le front de désactivation dû à la destruction des sites actifs se situe dans un plan perpendiculaire au sens de l'écoulement des fluides et progresse à peu près régulièrement d'amont en aval pendant la réaction.

En outre, au cours de l'opération de déchargement partiel du catalyseur, il est possible de déterminer avec encore plus de précision l'épaisseur de la couche du lit catalytique à remplacer en procédant par sondage à travers ce lit pour déterminer sur quelle distance, dans le sens de progression de la charge, le catalyseur est désactivé, par exemple, en déterminant la teneur en chlore d'échantillons de catalyseur prélevés à différentes distances de la face amont de la masse catalytique. En effet, on constate qu'immédiatement en aval du front de désactivation du catalyseur se manifeste une brusque augmentation de la teneur en chlore de ce catalyseur, de sorte que, par sondage, il est possible de déterminer l'épaisseur de la couche de catalyseur, en partant de la surface amont de celui-ci, à partir de laquelle la teneur en chlore de catalyseur commence à chuter d'une valeur prédéterminée, de 5 % par exemple.

Le catalyseur à remplacer peut être retiré du réacteur par tout moyen connu dans la technique, par exemple, en le laissant s'écouler par gravité hors du réacteur, dans le cas d'un réacteur fonctionnant à flux de charge ascendant, ou en l'aspirant à la partie supérieure du réacteur, dans le cas où celui-ci fonctionne à flux de charge descendant. Le réacteur sera ensuite rechargé, par le haut du réacteur, en catalyseur neuf ou en catalyseur régénéré à l'extérieur du réacteur, par tout moyen de chargement dense usuel, sous une atmosphère inerte, par exemple sous une atmosphère d'azote anhydre.

Comme indiqué ci-dessus, le processus d'isomérisation de la charge en $C_4$ ou en $C_5$-$C_6$ pourra alors être repris, éventuellement en inversant le sens de circulation de la charge, et l'on constatera que le taux d'isomérisation de l'effluent de l'unité d'isomérisation demeure sensiblement constant.

Le procédé conforme à l'invention pourra être mis en oeuvre sur un unique réacteur. Ce sera en particulier le cas pour des coupes à traiter en $C_4$ et, dans ce cas, du fait des problèmes entraînés par le chargement par le haut des réacteurs et l'inversion éventuelle du sens de circulation de la charge, après remplacement partiel du catalyseur, on utilisera de préférence un réacteur du type décrit dans FR-A-2 623 732.

Un unique réacteur peut également être utilisé pour l'isomérisation de charges en $C_5$-$C_6$, mais, pour de telles charges, il sera généralement préférable d'utiliser deux réacteurs disposés en série.

Dans cette forme de mise en oeuvre de l'invention, on mesurera le taux d'isomérisation des effluents du réacteur

disposé le plus en aval et, lorsque cet indice aura baissé d'une valeur prédéterminée comprise entre 10 et 30%, on interrompra le passage de la charge dans le réacteur disposé en amont et on remplacera par du catalyseur neuf ou régénéré une fraction déterminée du catalyseur disposée en amont de ce réacteur, avant d'y faire à nouveau circuler la charge, mais en inversant périodiquement le sens de circulation de celle-ci, après une phase de remplacement du catalyseur.

Pendant l'arrêt d'un réacteur, l'isomérisation de la charge est conduite sans interruption sur le seul autre réacteur et, après remplacement par du catalyseur neuf ou par du catalyseur régénéré d'une fraction disposée en amont de la masse catalytique du réacteur arrêté, on reprend le processus d'isomérisation, en inversant éventuellement de façon connue en soi le sens de la circulation de la charge à traiter, c'est-à-dire en lui faisant d'abord traverser le réacteur précédemment disposé en aval.

Le procédé d'isomérisation conforme à l'invention va être décrit ci-après de façon détaillée, en référence aux dessins annexés et aux exemples qui vont suivre. Sur les dessins :

La figure 1 est un schéma de principe d'une installation d'isomérisation à deux réacteurs d'une coupe de n-paraffines en $C_5$-$C_6$ en isoparaffines ;

La figure 2 est un diagramme schématique illustrant les variations en fonction du temps de l'indice d'octane moteur des effluents d'une telle installation, lorsque l'on épuise complètement le catalyseur du réacteur disposé en amont, avant de le remplacer par un catalyseur neuf ;

La figure 3 est un diagramme analogue à celui de la figure 2, montrant les faibles variations d'indice d'octane moteur des effluents de cette même installation, lorsque l'on utilise le procédé d'isomérisation conforme à la présente invention ;

Les figures 4, 5 et 6, sont des diagrammes relatifs aux essais qui sont décrits dans les exemples de mise en oeuvre de l'invention ;

La figure 7 illustre la mise en oeuvre de l'invention, dans le cas d'un unique réacteur d'isomérisation, par exemple pour l'isomérisation d'une coupe $C_4$.

Le dispositif schématisé sur la figure 1 est d'un type classique pour l'isomérisation de coupes de n-paraffines en $C_5$-$C_6$. Cette coupe arrive par la ligne 1, dans laquelle de l'hydrogène et éventuellement des gaz de recycle sont introduits par la ligne 2. Après être passée successivement dans deux échangeurs de chaleur, 3 et 4, à contre-courant des effluents des deux réacteurs d'isomérisation, la charge à isomériser est introduite dans le premier réacteur 5, ou réacteur amont, qui contient le catalyseur d'isomérisation. L'effluent de ce réacteur 5, après être passé dans l'échangeur 4, est ensuite introduit par la ligne 6 dans le second réacteur 7, ou réacteur aval, qui contient également du catalyseur d'isomérisation. Dans le cas présent, la charge à traiter circule de haut en bas dans les réacteurs 5 et 7, mais elle pourrait naturellement y circuler de bas en haut. L'effluent de la colonne 7, qui contient une charge enrichie en isoparaffines, est évacué par la ligne 8 et, après avoir traversé l'échangeur 3, est évacué vers un ensemble de séparation des produits, non représenté.

Le dispositif comprend des systèmes de vannes, non représentés, permettant d'interrompre indépendamment l'alimentation des réacteurs 5 et 7 et/ou d'inverser le sens de circulation de la charge.

Naturellement, des dispositifs de mesure de l'indice d'octane moteur de la charge à traiter et des effluents des réacteurs 5 et 7 sont également prévus.

Ainsi qu'il a été indiqué ci-dessus, compte tenu du fait que les catalyseurs "amorphes" utilisés sont coûteux et que les sites de Lewis et de Brönstedt ne sont pas régénérables, lorsqu'ils sont détruits par de l'eau, il est d'usage d'épuiser complètement la masse catalytique du réacteur amont 5, avant de la remplacer par une masse correspondante de catalyseur neuf ou régénéré, et d'inverser ensuite le sens de circulation de la charge à traiter, qui passe alors en premier lieu dans le réacteur 7, jusqu'à épuisement total du catalyseur contenu dans celui-ci. A chaque arrêt d'un réacteur pour le vider du catalyseur usé et le remplir de catalyseur neuf ou régénéré, la charge passe seulement à travers l'autre réacteur.

Si l'on mesure l'indice d'octane moteur MON de la charge traitée à la sortie du réacteur initialement disposé en amont (courbe $R_1$) et à la sortie du réacteur disposé initialement en aval (courbe $R_2$) en fonction du temps t, on constate (voir figure 2) que cet indice, à la sortie du réacteur disposé en aval, commence à baisser longtemps avant que la masse catalytique du réacteur disposé en amont ne soit remplacée. Ceci signifie que, tout au long de la mise en oeuvre du procédé, la masse catalytique du réacteur disposé à tour de rôle en aval commence à se désactiver avant le remplacement du catalyseur du réacteur disposé en amont.

L'indice d'octane moteur de la charge traitée sortant de l'unité (effluent du réacteur disposé en aval) présente donc une suite de maxima et de minima qui se traduisent par des pics sur les courbes de la figure 2. Ceci signifie que l'indice d'octane moteur des effluents de l'unité ne se maintient pas à son maximum, que la valorisation de la charge traitée n'est pas pleinement satisfaisante et que le bilan économique du procédé peut être amélioré.

C'est à ces inconvénients que vise à remédier le procédé de la présente invention, selon lequel on n'épuise pas en totalité la masse catalytique du réacteur disposé en amont avant de le remplacer, mais au contraire on remplace la fraction usée disposée en amont de cette masse, lorsque l'indice d'octane de la charge traitée, à la sortie de ce réacteur, baisse au-dessous d'une valeur donnée.

Comme on le voit sur la figure 3, qui est une figure analogue à la figure 2, dans le cas d'un procédé conforme à l'invention, il est ainsi possible de remplacer une fraction usée du catalyseur du réacteur amont avant que le catalyseur du réacteur aval ne commence à se désactiver ou ne se désactive de façon sensible. Il en résulte que l'indice d'octane moteur de la charge traitée reste pratiquement constante à sa valeur maximum, ce qui présente un avantage substantiel par rapport au procédé usuel.

La figure 7 illustre la mise en oeuvre de l'invention dans le cas où un unique réacteur d'isomérisation est utilisé. Dans ce cas, comme indiqué ci-dessus, on utilisera avantageusement un réacteur du type décrit dans FR-A-2 623 732.

La charge à traiter arrivant par la ligne 10 peut être introduite soit à la partie haute du réacteur 11 par la ligne 12, munie d'une vanne 13, soit à la partie basse du réacteur 11, par la ligne 14, munie d'une vanne 15.

La charge traitée est évacuée, respectivement, à la base du réacteur 11, par la ligne 16, munie d'une vanne 17, ou à la partie supérieure du réacteur, par la ligne 18, munie d'une vanne 19. Dans les deux cas, une ligne 20 achemine les effluents vers la partie inférieure d'une colonne de séparation 21, les isoparaffines étant évacuées par la ligne 22, à la partie supérieure de cette colonne, tandis que les n-paraffines résiduelles sont évacuées par la ligne 23, munie d'une vanne 24, à un niveau intermédiaire, et éventuellement recyclées à la ligne 10 d'alimentation du réacteur 11 par une ligne 25.

L'Exemple qui suit et qui n'a pas de caractère limitatif illustre l'application du procédé de l'invention à l'isomérisation d'une charge en $C_5$-$C_6$, à l'aide de deux réacteurs disposés en série.

## Exemple

On utilise une charge $C_5$-$C_6$, dont la composition en % en poids, est la suivante :

| | | | |
|---|---|---|---|
| - i $C_4$ | : | 0,00 | |
| - n $C_4$ | : | 0,36 | |
| - i $C_5$ | : | 20,05 | |
| - n $C_5$ | : | 38,50 | |
| - 22 D M $C_4$ | : | 1,40 | |
| - 23 D M $C_4$ | : | 2,28 | |
| - 2 M $C_5$ | : | 2,80 | |
| - 3 M $C_5$ | : | 11,89 | |
| - n $C_6$ | : | 7,32 | |
| - C $C_5$ | : | 11,81 | |
| - M C $C_5$ | : | 1,28 | ) naphtènes |
| - C $C_6$ | : | 0,16 | ) inhibiteurs de |
| - Benzène | : | 0,78 | ) réaction |
| - $C_7^+$ | : | 1,37 | (total 2,22 %) |

On utilise un dispositif d'isomérisation à deux réacteurs en série, du type représenté sur la figure 1, avec recycle à l'alimentation du fond de la colonne de séparation des effluents du réacteur aval (deuxième réacteur).

Les réacteurs contiennent chacun 25 tonnes d'un catalyseur préparé conformément au brevet européen N° 409 679 et présentant les caractéristiques suivantes : catalyseur acide monofonctionnel de type FRIEDEL-CRAFTS, support d'alumine traitée par le chlorure d'aluminium ou un autre dérivé organo aluminique de façon à constituer des sites

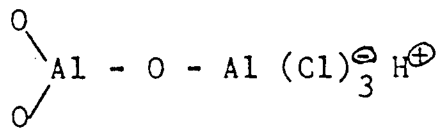

Les conditions opératoires sont les suivantes :

- débit de charge fraîche : 2 000t/j (soit une vitesse spatiale horaire de 2,8 volumes de charge par volume de catalyseur et par heure) ;
- débit de recycle en provenance du fond de la colonne de distillation des effluents : 400t/j.

On mesure le taux d'isomérisation total, défini comme

$$\frac{\text{isopentane}}{\text{pentane}} + \frac{\text{2-2-diméthylbutane}}{\text{hexane}},$$

par analyse chromatographique de la charge et des effluents des réacteurs.

On mesure également le nombre d'octane moteur (MON) des effluents des deux réacteurs.

Les conditions opératoires indiquées ci-dessus correspondent à un indice TIN de 94 et à un indice MON de 80 à la sortie du premier réacteur et à un indice TIN de 112 à la sortie du second réacteur.

L'indice TIN de l'isomérisat, après séparation des effluents du second réacteur est de 135 et l'indice MON de 87,5.

La figure 5 illustre la chute de l'indice MON des effluents du premier réacteur en fonction de la fraction désactivée, en % en poids, de la masse de catalyseur de ce réacteur. On constate qu'il existe une excellente corrélation, pratiquement linéaire, entre la chute du nombre d'octane moteur des effluents et la masse de catalyseur désactivée, puisqu'on note une chute du MON de 10 à 25 % lorsque la quantité de catalyseur désactivée passse de 33 à 66 % en poids.

Il est ainsi possible, en mesurant l'indice d'octane moteur des effluents du premier réacteur ou du second réacteur, suivant le sens de circulation de la charge à isomériser, de décider à quel moment il est opportun d'arrêter ces réacteurs et quelle quantité de catalyseur, disposée en amont dans ce réacteur, dans le sens de circulation de la charge, il faut déplacer.

Si l'on mesure par sondage dans ce même réacteur, avec un flux de charge descendant, la teneur en chlore du catalyseur en fonction de la distance avec le front amont du catalyseur, on constate (voir figure 6) que le front de désactivation du catalyseur se manifeste par une brusque augmentation de la teneur en chlore, qui se traduit par un palier sur la courbe relevée.

La Fig. 4, qui illustre l'évolution en fonction du temps du MON des effluents du réacteur aval du second réacteur, montre que la mise en oeuvre de l'invention a pour effet de majorer sensiblement le nombre d'octane moteur des effluents, qui passe de 78,5, sans les perfectionnements de l'invention, à 79,3, soit une différence de 0,8.

L'invention apporte donc un moyen simple et facile à mettre en oeuvre pour maintenir à un niveau élevé l'indice d'octane moteur des produits obtenus par isomérisation d'une coupe en $C_4$ ou en $C_5$-$C_6$.

**Revendications**

1. Procédé d'isomérisation de n-paraffines en isoparaffines d'une coupe d'hydrocarbures comprenant essentiellement soit des hydrocarbures à quatre atomes de carbone, soit des hydrocarbures à cinq et/ou six atomes de carbone, procédé dans lequel la coupe à traiter passe dans au moins un réacteur contenant un lit fixe de catalyseur que traverse la charge, ce catalyseur comprenant un support en oxyde minéral réfractaire, de 0,1 à 0,25 % en poids d'un métal de la mine du platine et de 2 à 10 % en poids de chlore, la réaction d'isomérisation étant conduite à une température de 100 à 200°C, sous une pression de 7 à 60 bars (7 à $60 \times 10^5$ Pascal), avec une vitesse spatiale horaire (volume de charge par volume de catalyseur et par heure) comprise entre 0,5 et 12, ce procédé étant caractérisé en ce que :

   - on mesure, de préférence en continu, le taux d'isomérisation des effluents de ce réacteur ou un paramètre directement lié à ce taux d'isomérisation ;
   - lorsque ce taux d'isomérisation a baissé d'une valeur prédéterminée, comprise entre 10 et 30%, on remplace par du catalyseur neuf ou régénéré une fraction disposée dans la partie amont de la masse catalytique du réacteur représentant entre un tiers et deux tiers de cette masse catalytique.

- lorsque l'opération de remplacement de la fraction amont du catalyseur du réacteur est terminée, on fait à nouveau traverser ce réacteur à la charge.

2. Procédé selon la revendication 1, caractérisé en ce que, lorsque le taux d'isomérisation de la charge traitée ou un paramètre directement lié à ce taux d'isomérisation a baissé de 10%, on remplace le tiers de la masse catalytique de ce réacteur disposé en amont.

3. Procédé selon l'une des revendication 1 et 2, caractérisé en ce que, lorsque le taux d'isomérisation des effluents de la charge traitée ou un paramètre directement lié à ce taux d'isomérisation a baissé de 15 à 20%, on remplace la moitié de la masse catalytique de ce réacteur disposée en amont.

4. Procédé selon la revendication 1, caractérisé en ce que, lorsque le taux d'isomérisation de la charge traitée ou un paramètre directement lié à ce taux d'isomérisation a baissé de 30%, on remplace les 60 à 75% de la masse catalytique du réacteur disposés en amont.

5. Procédé selon l'une des revendications 1 à 4, caractérisé en ce que le taux d'isomérisation des effluents du réacteur est déterminé par analyse chromatographique de la charge et des effluents.

6. Procédé selon l'une des revendications 1 à 4, caractérisé en ce que le taux d'isomérisation des effluents du réacteur est déterminé par mesure du nombre d'octane moteur de la charge et des effluents.

7. Procédé selon l'une des revendications 1 à 6, caractérisé en ce que la fraction amont de la masse catalytique à remplacer par du catalyseur neuf ou régénéré est déterminée par sondage dans cette masse catalytique à partir de son extrémité amont.

8. Procédé selon la revendication 7, caractérisé en ce que le sondage de la masse catalytique du réacteur porte sur la teneur en chlore du catalyseur.

9. Procédé selon la revendication 8, caractérisé en ce que l'on remplace la fraction du catalyseur du réacteur disposée en amont d'une zone où la teneur en chlore de ce catalyseur varie brusquement d'une valeur prédéterminée, par exemple d'environ 5 %.

10. Procédé selon l'une des revendications 1 à 9, caractérisé en ce que l'on utilise un unique réacteur d'isomérisation.

11. Procédé selon l'une des revendications 1 à 10, caractérisé en ce que le ou les réacteurs sont chargés par la méthode dite de "chargement dense".

12. Procédé selon l'une des revendications 1 à 10, caractérisé en ce que :

    - l'on utilise deux réacteurs d'isomérisation disposés en série, le taux d'isomérisation ou un paramètre directement lié à ce taux étant mesuré dans les effluents du réacteur disposé le plus en aval ;
    - on interrompt le passage de la charge dans le réacteur disposé en amont, lorsque le taux d'isomération mesuré ou un paramètre lié à ce taux a baissé d'une valeur prédéterminée comprise entre 10 et 30% ;
    - on remplace une fraction déterminée du catalyseur disposée dans la partie amont du réacteur amont ;
    - on fait à nouveau circuler la charge dans les deux réacteurs, en inversant périodiquement son sens de circulation, après une phase de remplacement du catalyseur.

**Claims**

1. A process for the isomerisation of n-paraffins into isoparaffins from a hydrocarbon fraction substantially comprising either hyrocarbons with four carbon atoms, or hydrocarbons with five and/or six carbon atoms, in which process the fraction to be treated passes into at least one reactor containing a fixed bed catalyst, through which the charge passes, said catalyst comprising a support of refractory mineral oxide, from 0.1 to 0.25 % by weight of a metal of the platinum type and from 2 to 10 % by weight of chlorine, the isomerisation reaction being carried out at a temperature of 100 to 200°C, at a pressure of 7 to 60 bar (7 to $60/10^5$ Pascal), with an hourly spatial velocity (volume of charge per volume of catalyst per hour) of between 0.5 and 12, said process being characterised:

    - by measuring, preferably continuously, the rate of isomerisation of the effluents from the reactor or a parameter directly associated with the isomerisation rate;

- by replacing with new or regenerated catalyst, when the isomerisation rate has decreased by a predetermined value, of between 10 and 30 %, a fraction disposed in the upstream part of the catalytic mass of the reactor, representing between one third and two thirds of the catalytic mass;
- in that when the operation of replacing the upstream fraction of catalyst in the reactor has ended, the charge is again induced to pass through the reactor.

2. A process according to claim 1, characterised in that, when the isomerisation rate of the treated charge or a parameter directly associated with the isomerisation rate has decreased by 10 %, the third of the catalytic mass of the reactor disposed upstream is replaced.

3. A process according to either of claims 1 and 2, characterised in that, when the isomerisation rate of the effluents of the treated charge or a parameter directly associated with the isomerisation rate has decreased by 15 to 20 %, the half of the catalytic mass of the reactor disposed upstream is replaced.

4. A process according to claim 1, characterised in that, when the isomerisation rate of the treated charge or a parameter directly associated with the isomerisation rate has decreased by 30 %, 60 to 75 % of the catalytic mass of the reactor disposed upstream is replaced.

5. A process according to any one of claims 1 to 4, characterised in that the isomerisation rate of the effluents from the reactor is determined by chromatographic analysis of the charge and of the effluents.

6. A process according to any one of claims 1 to 4, characterised in that the isomerisation rate of the effluents from the reactor is determined by measuring the octane number of the charge and of the effluents.

7. A process according to any one of claims 1 to 6, characterised in that the upstream fraction of the catalytic mass to be replaced with new or regenerated catalyst is determined by sampling of said catalytic mass starting from its upstream end.

8. A process according to claim 7, characterised in that the sampling of the catalytic mass of the reactor relates to the chlorine content of the catalyst.

9. A process according to claim 8, characterised by replacing the fraction of the catalyst of the reactor disposed upstream of a zone in which the chlorine content of the catalyst varies abruptly from a predetermined value, for example by approximately 5 %.

10. A process according to any one of claims 1 to 9, characterised in that the a single isomerisation reactor is used.

11. A process according to any one of claims 1 to 10, characterised in that the reactor or reactors are charged using the so-called "dense charging" method.

12. A process according to any one of claims 1 to 10, characterised in that:

- two isomerisation reactors arranged in series are used, the isomerisation rate or a parameter directly associated with the isomerisation rate being measured in the effluents from the reactor disposed furthest downstream;
- the passage of the charge into the upstream reactor is interrupted when the isomerisation rate measured or a parameter directly associated with the isomerisation rate has decreased by a predetermined value of between 10 and 30 %;
- a given fraction of the catalyst disposed in the upstream part of the reactor is replaced;
- the charge is caused to circulate again in the two reactors, by periodically reversing its circulation direction, after a catalyst replacement stage.

**Patentansprüche**

1. Verfahren zur Isomerisation von n-Paraffinen in Isoparaffine einer Fraktion von Kohlenwasserstoffen, welche im wesentlichen aus Kohlenwasserstoffen mit vier Kohlenstoffatomen oder Kohlenwasserstoffen mit fünf und/oder sechs Kohlenstoffatomen besteht, wobei die zu behandelnde Fraktion mindestens einen Reaktor durchläuft, der ein von der Charge durchquertes Festbett eines Katalysators enthält, welcher einen Träger aus feuerfestem mineralischen Oxyd, 0,1 bis 0,25 Gewichtsprozent eines Platinmetalls und 2 bis 10 Gewichtsprozent Chlor aufweist, und

wobei die Isomerisationsreaktion bei einer Temperatur von 100 bis 200° C unter einem Druck von 7 bis 60 bar ( 7 bis 60 • $10^5$ Pascal) mit einer stündlichen Volumengeschwindigkeit ( Chargenvolumen pro Katalysatorvolumen und pro Stunde ) zwischen 0,5 und 12 durchgeführt wird, dadurch **gekennzeichnet**, daß

das Isomerisationsverhältnis der Abgänge dieses Reaktors oder ein mit diesem Isomerisationsverhältnis direkt verknüpfter Parameter gemessen wird, vorzugsweise kontinuierlich,

ein im stromaufwärts gelegenen Teil der katalytischen Masse des Reaktors angeordneter Bruchteil, welcher zwischen einem Drittel und zwei Dritteln dieser katalytischen Masse darstellt, durch neuen oder regenerierten Katalysator ersetzt wird, sobald das Isomerisationsverhältnis um einen vorgegebenen Wert zwischen 10 und 30 % abgefallen ist,

der Reaktor wieder von der Charge durchlaufen gelassen wird, sobald der Vorgang des Ersetzens des stromaufwärts gelegenen Bruchteils des Katalysators des Reaktors beendet ist.

**2.** Verfahren nach Anspruch 1, dadurch **gekennzeichnet**, daß das stromaufwärts gelegene Drittel der katalytischen Masse des Reaktors ersetzt wird, sobald das Isomerisationsverhältnis der behandelten Charge oder ein damit direkt verknüpfter Parameter um 10 % abgefallen ist.

**3.** Verfahren nach Anspruch 1 oder 2, dadurch **gekennzeichnet**, daß die stromaufwärts gelegene Hälfte der katalytischen Masse des Reaktors ersetzt wird, sobald das Isomerisationsverhältnis der Abgänge der behandelten Charge oder ein damit direkt verknüpfter Parameter um 15 bis 20 % abgefallen ist.

**4.** Verfahren nach Anspruch 1, dadurch **gekennzeichnet**, daß die stromaufwärts gelegenen 60 bis 75 % der katalytischen Masse des Reaktors ersetzt werden, sobald das Isomerisationsverhältnis der behandelten Charge oder ein damit direkt verknüpfter Parameter um 30 % abgefallen ist.

**5.** Verfahren nach einem der Ansprüche 1 bis 4, dadurch **gekenzeichnet**, daß das Isomerisationsverhältnis der Abgänge des Reaktors durch chromatografische Analyse der Charge und der Abgänge bestimmt wird.

**6.** Verfahren nach einem der Ansprüche 1 bis 4, dadurch **gekennzeichnet**, daß das Isomerisationsverhältnis der Abgänge des Reaktors durch Messung der Motoroktanzahl der Charge und der Abgänge bestimmt wird.

**7.** Verfahren nach einem der Ansprüche 1 bis 6, dadurch **gekennzeichnet**, daß der durch neuen oder regenerierten Katalysator zu ersetzende stromaufwärts gelegene Bruchteil der katalytischen Masse durch Sondierung in dieser katalytischen Masse von ihrem stromaufwärts gelegenen Ende her bestimmt wird.

**8.** Verfahren nach Anspruch 7, dadurch **gekennzeichnet**, daß die Sondierung der katalytischen Masse des Reaktors sich auf den Chlorgehalt des Katalysators bezieht.

**9.** Verfahren nach Anspruch 8, dadurch **gekennzeichnet**, daß derjenige Bruchteil des Katalysators des Reaktors ersetzt wird, welcher stromaufwärts von einer Zone angeordnet ist, wo der Chlorgehalt dieses Katalysators sich brüsk um einen vorgegebenen Wert von beispielsweise etwa 5 % ändert.

**10.** Verfahren nach einem der Ansprüche 1 bis 9, dadurch **gekennzeichnet**, daß ein einziger Isomerisationsreaktor benutzt wird.

**11.** Verfahren nach einem der Ansprüche 1 bis 10, dadurch **gekennzeichnet**, daß der oder die Reaktoren nach der sogenannten " dichten Beschickungsmethode " beschickt wird bzw. werden.

**12.** Verfahren nach einem der Ansprüche 1 bis 10, dadurch **gekennzeichnet**, daß

zwei in Reihe angeordnete Isomerisationsreaktoren benutzt werden, wobei das Isomerisationsverhältnis oder ein damit direkt verknüpfter Parameter in den Abgängen des am meisten stromabwärts gelegenen Reaktors gemessen wird,

der Durchlauf der Charge durch den stromaufwärts angeordneten Reaktor unterbrochen wird, sobald das gemessene Isomerisationsverhältnis oder der gemessene damit direkt verknüpfte Parameter um einen vorgegebenen Wert zwischen 10 und 30 % abgefallen ist,

ein im stromaufwärts gelegenen Teil des stromaufwärts gelegenen Reaktors angeordneter, vorgegebener Bruchteil des Katalysators ersetzt wird,

die Charge wieder in den beiden Reaktoren umlaufen gelassen wird, wobei ihre Umlaufrichtung nach einer Phase des Ersatzes des Katalysators periodisch umgekehrt wird.

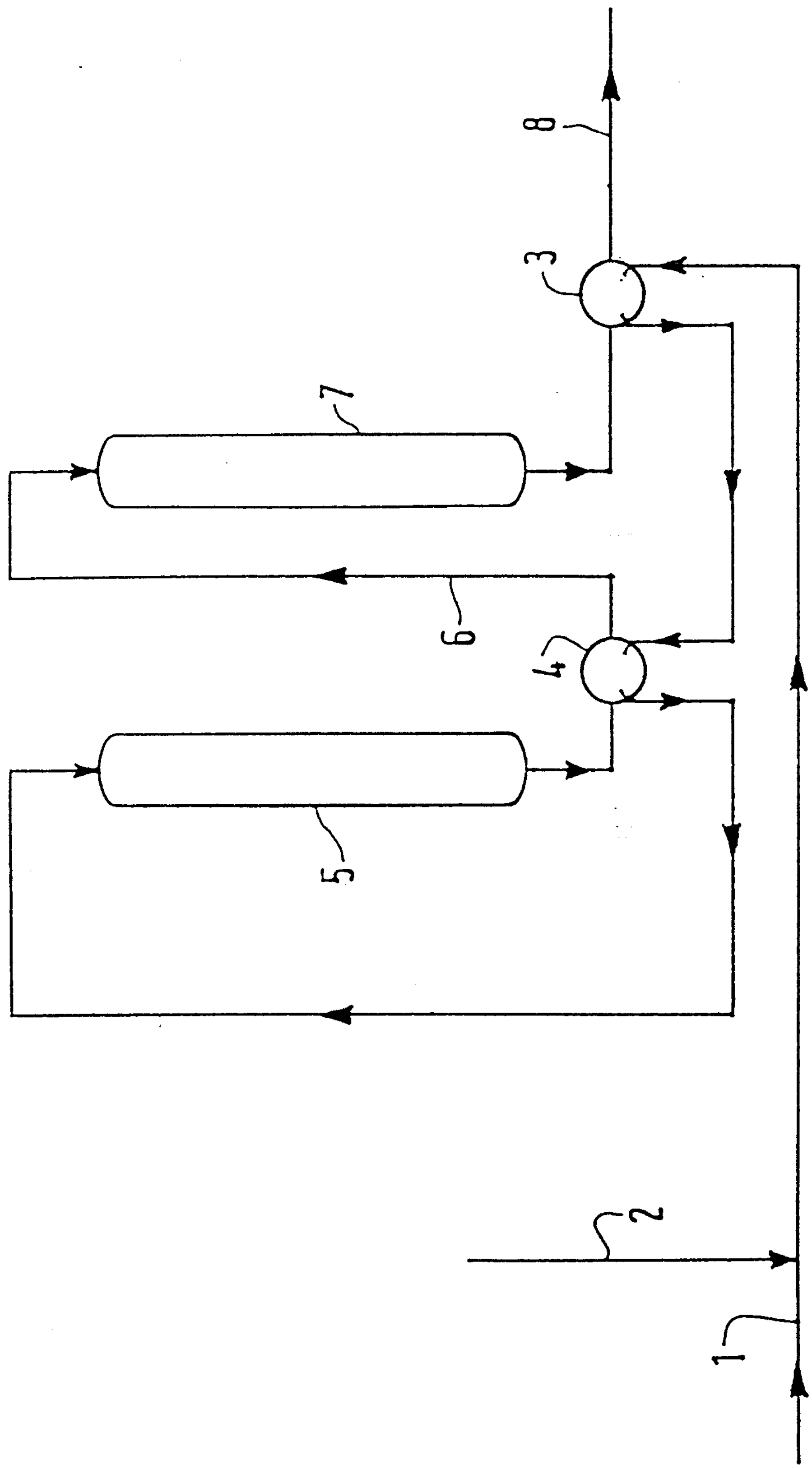


FIG.1

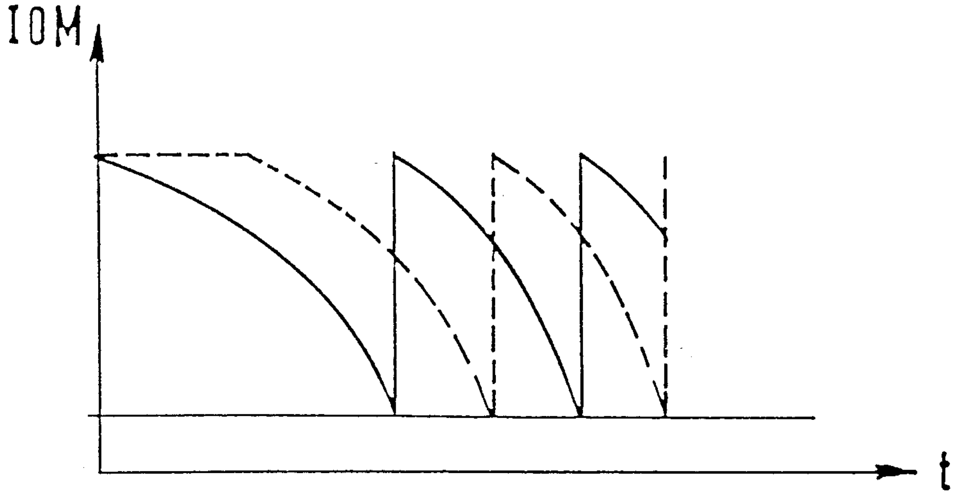


FIG.2

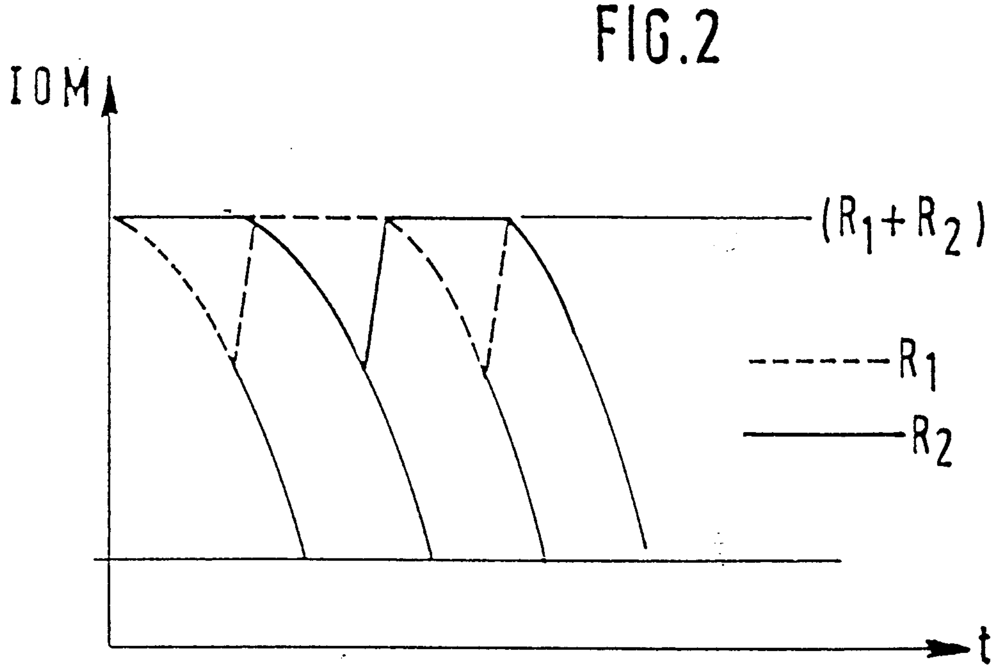


FIG.3

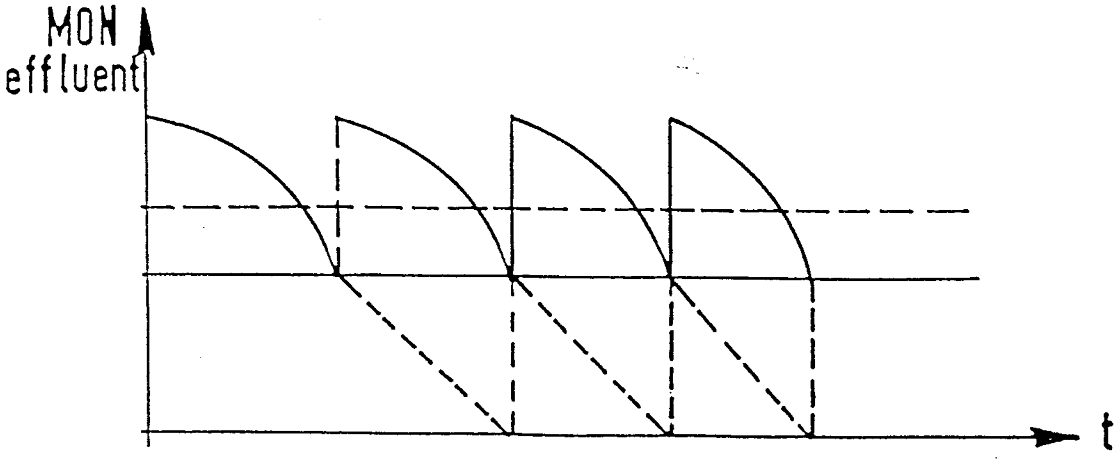


FIG.4

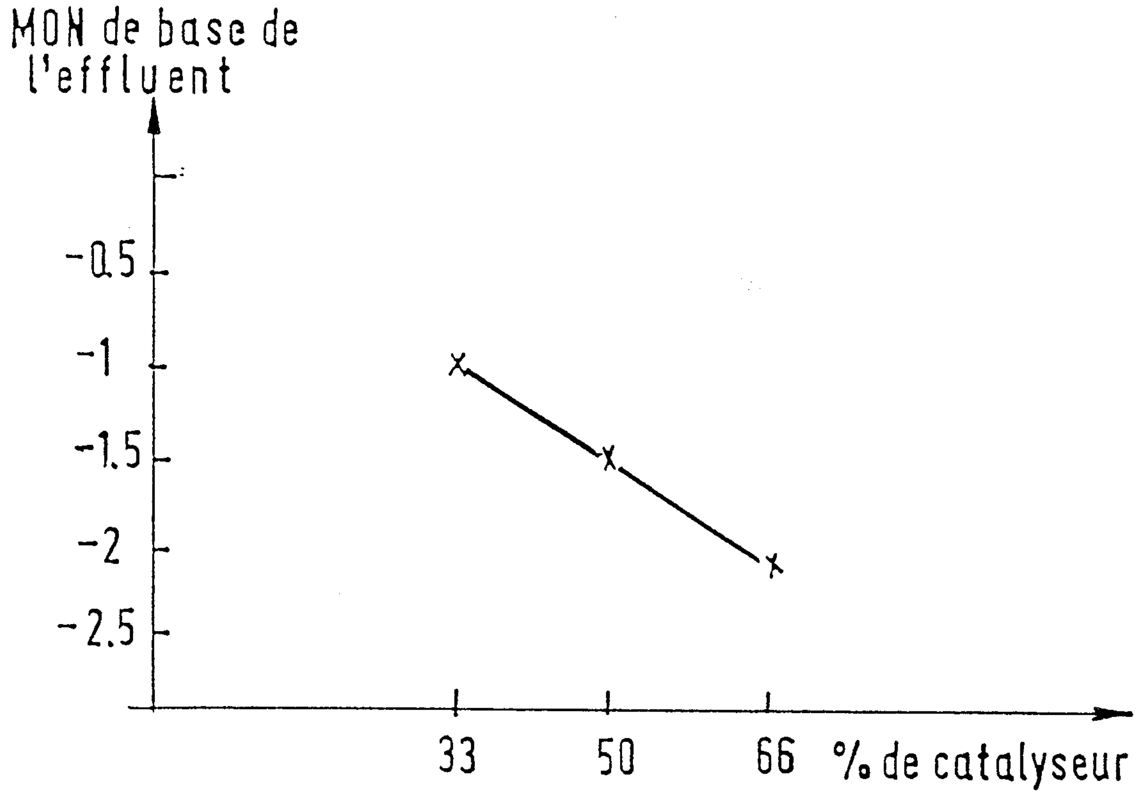
MON de base de
l'effluent
-0.5
-1
-1.5
-2
-2.5
33
50
66
% de catalyseur

FIG.5

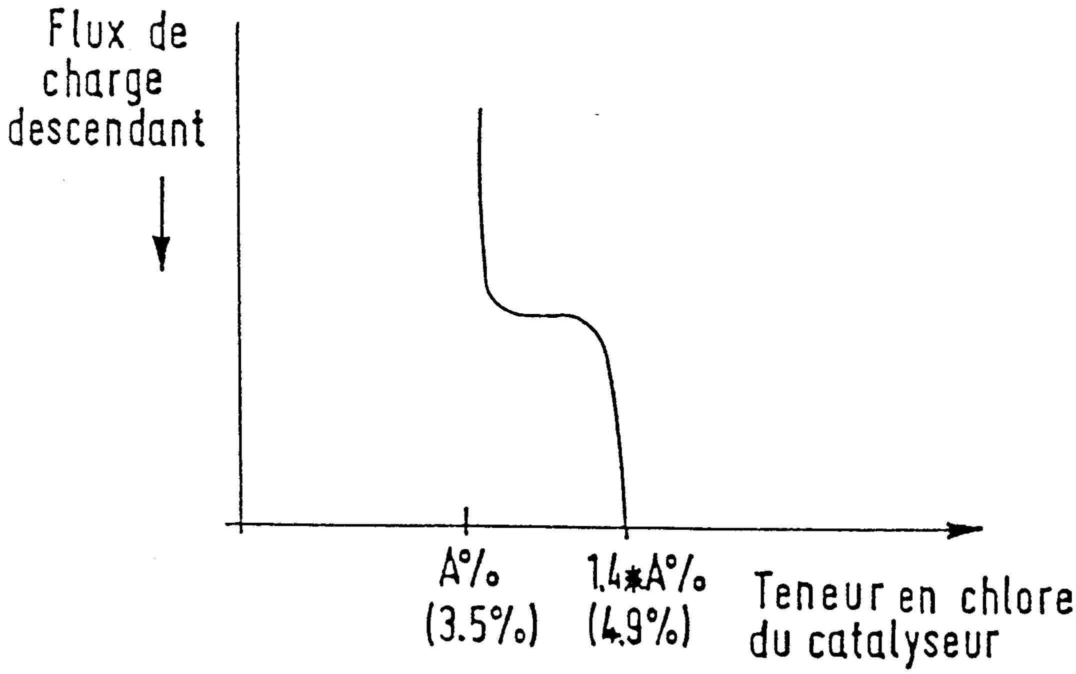
Flux de
charge
descendant
A%
(3.5%)
1.4*A%
(4.9%)
Teneur en chlore
du catalyseur

FIG.6

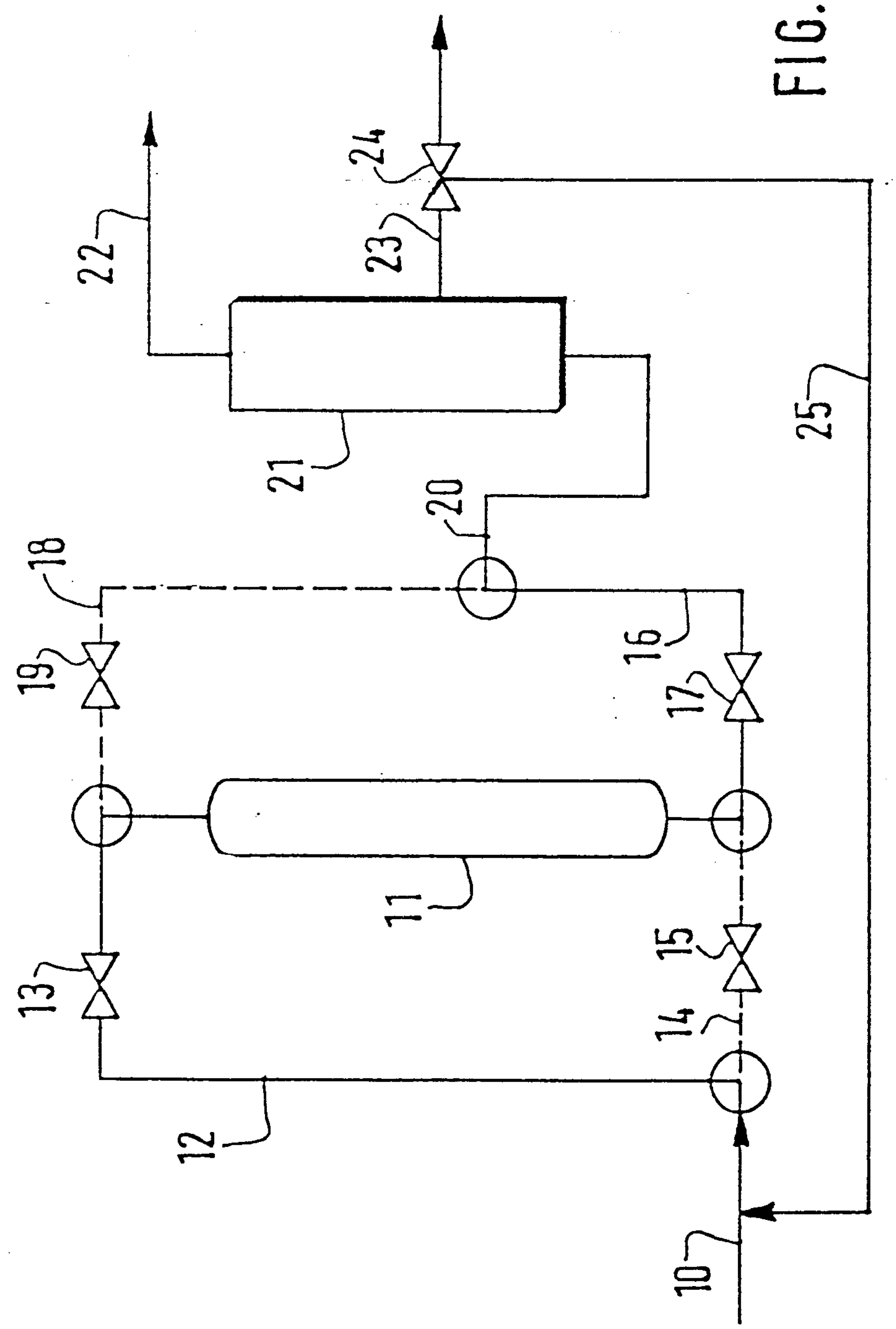


FIG.7